(19) Europäisches Patentamt
European Patent Office
Office européen des brevets

(11) Veröffentlichungsnummer : **0 134 479**
**B1**

(12) # EUROPÄISCHE PATENTSCHRIFT

(45) Veröffentlichungstag der Patentschrift : 02.03.88

(21) Anmeldenummer : 84107861.1

(22) Anmeldetag : 05.07.84

**ERRATUM**

| DIE TEXTSTELLE :<br>TEXT PUBLISHED :<br>LE PASSAGE SUIVANT : | (SEITE, SPALTE, ZEILE)<br>(PAGE, COLUMN, LINE)<br>(PAGE,COLONNE,LIGNE) | | LAUTET BERICHTIGT:<br>SHOULD READ :<br>DEVRAIT ETRE LU : |
|---|---|---|---|
| Âl | 22 | 35<br>36<br>37<br>38 | µl |

Tag der Entscheidung über die Berichtigung
Date of decision on rectification:
Date de décision portant sur modification: 20.07.88

Ausgabe- und Veröffentlichungstag:
Issue and publication date:
Date d'edition et de publication: 14.09.88

Patbl.Nr: 88/37
EPB no:
Bull. no:

(19) Europäisches Patentamt
European Patent Office
Office européen des brevets

(11) Veröffentlichungsnummer : **0 134 479**
**B1**

(12) # EUROPÄISCHE PATENTSCHRIFT

(45) Veröffentlichungstag der Patentschrift : **02.03.88**

(51) Int. Cl.⁴ : **C 07 K 7/10**, C 12 P 21/06, C 12 N 9/99

(21) Anmeldenummer : **84107861.1**

(22) Anmeldetag : **05.07.84**

(54) **Modifizierte Protease-Inhibitoren, Verfahren zu ihrer Herstellung und daraus bereitete pharmazeutische Mittel.**

(30) Priorität : **07.07.83 DE 3324534**

(43) Veröffentlichungstag der Anmeldung : **20.03.85 Patentblatt 85/12**

(45) Bekanntmachung des Hinweises auf die Patenterteilung : **02.03.88 Patentblatt 88/09**

(84) Benannte Vertragsstaaten : **AT BE CH DE FR GB IT LI LU NL SE**

(56) Entgegenhaltungen :
**DE-A- 2 808 396**
**HOPPE-SEYLER'S ZEITSCHRIFT FÜR PHYSIOLOGISCHE CHEMIE, Band 361, 1980; U. SEEMÜLLER et al. "Structure of the Elastase-Cathepsin G Inhibitor of the Leech Hirudo medicinalis", Seiten 1841-1846**
**HOUBEN-WEYL, Methoden der organischen Chemie, Band XV/1, Stuttgart 1974**
**A. Baici, U. Seemüller, Biochem. J. (1984), Band 218, Seiten 829-833**

(73) Patentinhaber : **PLANTORGAN WERK Heinrich G.E. Christensen KG**
**Hornbusch 1**
**D-2903 Bad Zwischenahn (DE)**

**CIBA-GEIGY AG**
**Klybeckstrasse 141**
**CH-4002 Basel (CH)**

(72) Erfinder : **Seemüller, Ursula, Dr.**
**Destouchesstrasse 60**
**D-8000 München 40 (DE)**
Erfinder : **Dodt, Johannes**
**Aberlestrasse 4**
**D-8000 München 70 (DE)**
Erfinder : **Fritz, Hans, Prof. Dr.**
**Neulingerstrasse 15**
**D-8011 Hohenbrunn (DE)**

(74) Vertreter : **Kinzebach, Werner, Dr.**
**Patentanwälte Reitstötter, Kinzebach und Partner**
**Sternwartstrasse 4 Postfach 86 06 49**
**D-8000 München 86 (DE)**

**EP 0 134 479 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue modifizierte Protease-Inhibitoren mit der Primärstruktur von Eglin B und C, Verfahren zu ihrer Herstellung und pharmazeutische Präparate, die diese neuen Verbindungen enthalten.

Es ist bekannt, dass Extrakte aus Blutegeln Protease-Inhibitoren enthalten. Die DE-OS 2 808 396 beschreibt zwei Protease-Inhibitoren, die mit den wissenschaftlichen Namen Eglin B und Eglin C bezeichnet wurden. Diese weisen eine starke Hemmwirkung für Chymotrypsin, Subtilisin, PMN-Granulozytenelastase und Kathepsin G auf, dagegen hemmen sie Trypsin und Pankreaselastase nur schwach. Beide Egline bestehen aus 70 Aminosäureresten. Sie sind identisch in ihren N-Termini (Thr) und weisen gleiche C-terminale Sequenzen auf (-Val-Gly).

Die Egline gehören zu den stärksten heute bekannten Hemmern von PMN-Granulozytenelastase. PMN-Granulozytenelastase, eine neutrale Protease, ist beim Abbau von Geweben und löslichen Proteinen (z. B. Blutgerinnungsfaktoren, Komplement-Faktoren etc.) beteiligt. Unkontrollierte Freisetzung dieser Protease(n) im Organismus kann einen Entzündungsvorgang verstärken und Gewebeabbau durch unspezifische Proteolyse hervorrufen. Auf Grund ihrer Eigenschaften sind die Egline daher für die Anwendung in der medizinischen Therapie (Antiinflammation, Antiphlogistik, septischer Schock, Lungenemphysem, Mucoviscidose etc.) von grossem Interesse.

Der Erfindung liegt die Aufgabe zugrunde, auf der Grundlage von Eglin B und C modifizierte Protease-Inhibitoren mit vereinfachter Struktur und ähnlicher Wirksamkeit wie die natürlichen, nicht modifizierten Produkte zu schaffen.

Gegenstand der Erfindung sind modifizierte Protease-Inhibitoren mit der Primärstruktur von Eglin B und C, die dadurch gekennzeichnet sind, dass die Primärstruktur um 2 bis 10 Aminosäurebausteine am Anfang des Moleküls (N-Terminus) und/oder 2 bis 6 Aminosäurebausteine am Ende des Moleküls (C-Terminus) verkürzt ist.

Insbesondere sind modifizierte Protease-Inhibitoren mit der Primärstruktur von Eglin B und Eglin C dadurch gekennzeichnet, dass die Primärstruktur um 2 bis 6 Aminosäurebausteine am Anfang des Moleküls (N-Terminus) und/oder 2 Aminosäurebausteine am Ende des Moleküls (C-Terminus) verkürzt ist.

Vorteilhafte modifizierte Protease-Inhibitoren mit der Primärstruktur von Eglin C sind dadurch gekennzeichnet, dass die Primärstruktur um 4 bis 6 Aminosäurebausteine am Anfang des Moleküls (N-Terminus) und/oder 2 Aminosäurebausteine am Ende des Moleküls (C-Terminus) verkürzt ist.

Vorzugsweise handelt es sich dabei um den modifizierten Protease-Inhibitor F1, der durch folgende Primärstruktur:

H-Ser-Glu-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

und eine Dissoziationskonstante $K_i = 5.52 \times 10^{-11}$ mol/l für einen Chymotrypsin-Komplex (Substrat: SucAlaAlaProPhepNA) gekennzeichnet ist, und um den modifizierten Protease-Inhibitor F2, der durch folgende Primärstruktur:

H-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

und eine Dissoziationskonstante $K_i = 5.37 \times 10^{-11}$ mol/l für einen Chymotrypsin-Komplex (Substrat: SucAlaAlaProPhepNA) gekennzeichnet ist.

Die erfindungsgemässen modifizierten Inhibitoren bilden mit Chymotrypsin Komplexverbindungen, sie inhibieren also die Protease Chymotrypsin. Wie aus Tabelle 1 hervorgeht, sind die Dissoziationskonstanten von Komplexverbindungen erfindungsgemässer Verbindungen mit denen von Eglin C vergleichbar.

Tabelle 1

| | $K_i$ (mol/l) | inhibierte Protease |
|---|---|---|
| Eglin C | $8 \times 10^{-10}$ a) | leukozytäre Elastase |
| | $5 \times 10^{-11}$ | leukozytäres Kathepsin G |
| Modifizierungsprodukt F 1 | $5.52 \times 10^{-11}$ b) | Chymotrypsin |
| Modifizierungsprodukt F 2 | $5.37 \times 10^{-11}$ b) | Chymotrypsin |

a) Substrat: SucAlaAlaProValNMec, b) Substrat: SucAlaAlaProPhepNA

Die Dissoziationskonstanten sind nach der bekannten Methode von N. M. Green und E. Work bestimmt, die im Biochemical Journal 54, 347 bis 352 beschrieben ist.

Bei den erfindungsgemässen verkürzten Eglinen handelt es sich um modifizierte Produkte, deren Hemmaktivität überraschenderweise vollständig erhalten geblieben ist.

Die erfindungsgemässen Protease-Inhibitoren zeichnen sich durch eine Reihe von Vorteilen aus. Die im Vergleich zu den Eglinen verkürzte Peptidkette führt dazu, dass sie leichter zu synthetisieren sind, ferner wirken sie weniger allergisierend und weisen eine bessere Resorbierbarkeit auf.

Die Verbindungen dieser Erfindung lassen sich z. B. durch enzymatischen Abbau von Eglin B oder C oder durch chemische Synthese erhalten.

Ein erfindungsgemässer enzymatischer Abbau von Eglin B oder C erfolgt in Form einer limitierten Proteolyse. Dazu verwendet man Peptidasen, wie Carboxypeptidasen, d. h. Proteasen, die eine Aminosäurekette vom Carboxylende her spalten und/oder Aminopeptidasen, d. h. Proteasen, die eine Aminosäurekette vom Aminoende her angreifen. Für das erfindungsgemässe Verfahren geeignete Proteasen, die an Träger gebunden sein können, sind Carboxypeptidase A, Leucinaminopeptidase und die Kathepsine A, B, C, D ; insbesondere sind jedoch Carboxypeptidase Y und Kathepsin C geeignet. Dabei spaltet Kathepsin C als Dipeptidylaminopeptidase sequentiell Dipeptide vom unsubstituierten Aminoende von Proteinen ab. Die Wirkungsweise von Kathepsin ist aus der folgenden Gegenüberstellung der Aminosäuresequenzausschnitte (beginnend mit dem N-Terminus) von Eglin C und den durch Abbau mit Kathepsin C erhältlichen Modifizierungsprodukten F1 und F2 ersichtlich :

| | | | | | | | | | | | | | | |
|---|---|---|---|---|---|---|---|---|---|---|---|---|---|---|
| Eglin C : | Thr | Glu | Phe | Gly | Ser | Glu | Leu | Lys | Ser | Phe | Pro | Glu | Val | Val... |
| F1 : | | | | | Ser | Glu | Leu | Lys | Ser | Phe | Pro | Glu | Val | Val... |
| F2 : | | | | | | | Leu | Lys | Ser | Phe | Pro | Glu | Val | Val... |

Dieses Verfahren zur Herstellung von modifizierten Proteaseinhibitoren durch limitierte Proteolyse ist dadurch gekennzeichnet, dass man

a) Eglin B oder C in ein geeignetes Puffersystem von pH 4 bis 7 einbringt,

b) mit einer, in einem geeigneten Puffersystem gelösten Peptidase und gegebenenfalls mit 0.5 % Natriumlaurylsulfat versetzt,

c) 2 bis 72 Stunden bei 10 bis 40 °C inkubiert,

d) das Reaktionsgemisch chromatographisch auftrennt und

e) entsalzt.

Das Verfahren zur Herstellung des modifizierten Inhibitors F1 ist vorzugsweise dadurch gekennzeichnet, dass man

a) Eglin C in 1 %-iger Essigsäure löst,

b) diese Lösung mit einer Lösung von Kathepsin C (20 U/l · 14 ml) in einem Puffersystem aus 4 %-igem Pyridin, Wasser, 0.5 %-iger Essigsäure, 0.1 N HCl, 0.375 M 2-Mercaptoethanol, 0.2 M EDTA versetzt und den pH auf 5.0 einstellt,

c) 4 Stunden bei 37 °C inkubiert und

d) die Reaktionsmischung an einem Kationenaustauscher Sephadex® SP-C25 auftrennt, indem man mit 0.05 M Ammoniumacetat von pH 5.0 äquilibriert und mit einem Gradienten von pH 5.0, hergestellt aus gleichen Volumenteilen 0.05 M Ammoniumacetat und 0.4 M Natriumchlorid in 0.05 M Ammoniumacetat eluiert.

Das Verfahren zur Herstellung des modifizierten Inhibitors F2 ist vorzugsweise dadurch gekennzeichnet, dass man

a) Eglin C in 1 %-iger Essigsäure löst,

b) diese Lösung mit einer Lösung von Kathepsin C (20 U/l · 14 ml) in einem Puffersystem aus 4 %-igem Pyridin, Wasser, 0,5 %-iger Essigsäure, 0.1 N Salzsäure, 0.375 M 2-Mercaptoethanol, 0.2 M EDTA versetzt und den pH auf 5.0 einstellt,

c) 48 Stunden bei 37 °C inkubiert und

d) die Reaktionsmischung an einem Kationenaustauscher Sephadex® SP-C25 auftrennt, indem man mit 0.05 M Ammoniumacetat vom pH 5.0 äquilibriert und mit einem Gradienten von pH 5.0, hergestellt aus gleichen Volumenteilen 0.05 M Ammoniumacetat und 0.4 M Natrium chlorid in 0.05 M Ammoniumacetat eluiert, wobei zuerst der modifizierte Protease-Inhibitor F1, dann der modifizierte Protease-Inhibitor F2 eluiert wird.

Es ist verständlich, dass die Inkubationszeit massgebend für den Grad des Abbaus des Eglin-Moleküls ist. Beispielsweise werden beim Abbau des Eglin C mit Kathepsin C nach einer Inkubationszeit von 4 Stunden ausschliesslich das Modifizierungsprodukt F1, nach 24 Stunden das Modifizierungsprodukt F1 zusammen mit dem Modifizierungsprodukt F2 und nach 48 Stunden überwiegend das Modifizierungsprodukt F2 erhalten. Der Reaktionsverlauf kann mit Hilfe der Disk-Elektrophorese (pH 8.9 ;

15 %-iges Gel ; entsprechend einem Maurer-System Nr. 2) verfolgt werden, dabei werden folgende $R_f$-Werte erhalten :

| | |
|---|---|
| Eglin C : | 0.52 |
| Modifizierungsprodukt F1: | 0.42 |
| Modifizierungsprodukt F2: | 0.3 |

Die beiden wie vorstehend beschrieben erhaltenen modifizierten Inhibitoren F1 und F2 sind — wie durch diskontinuierliche Gelelektrophorese (in einem Gelsystem Nr. 2 nach Maurer) festgestellt wurde — elektrophoretisch einheitlich.

Ferner lassen sich die Verbindungen dieser Erfindung auf chemischem Wege erhalten. Eine chemische Synthese kann unter Verwendung entsprechender, gegebenenfalls sinnvoll geschützter Aminosäurebausteine und auf nach dem Stand der Technik bekannte Weise erfolgen. Insbesondere ist eine solche Peptidsynthese dadurch gekennzeichnet, dass man in einer den erfindungsgemässen Verbindungen entsprechenden Verbindung, in welcher mindestens eine von vorhandenen Amino-, Hydroxy- und/oder Carboxygruppen eine Schutzgruppe trägt, vorhandene Schutzgruppen abspaltet. Zur näheren Erläuterung einer solchen Synthese dienen die nachfolgenden Syntheseschemata, die beispielhaft die Synthese der Verbindungen F1 und F2 beschreiben : Die in der Beschreibung und im Anspruchssatz verwendeten Abkürzungen haben die folgende Bedeutung, vgl. auch Verzeichnis von Abkürzungen in Houben-Weyl, Band 15/1 ; Synthese von Peptiden, Georg Thieme Verlag, Stuttgart, 1974 :

BOC-tert.-Butyloxycarbonyl
BPOC-2-[Biphenylyl-(4)]-propyl(2)-oxycarbonyl
But-tert.-Butyl
DCCI-Dicyclohexylcarbodiimid
EDTA-Ethylendiamintetraessigsäure
HOBt-N-Hydroxybenzotriazol
Me-Methyl
Mec-4-Methyl-cumar-7-yl
ONP-4-Nitrophenylester
pNA-p-Nitroanilid
Suc-Succinyl
Z-Benzyloxycarbonyl
SucAlaAlaProPhepNA = Succinyl-L-Alanyl-L-Alanyl-L-Prolyl-L-Phenylalanyl-p-nitroanilid
SucAlaAlaProValNMec = Succinyl-L-Alanyl-L-Alanyl-L-Prolyl-L-Valyl-7-Amino-4-methylcumarylamid

1. Synthese des modifizierten Protease-Inhibitors F1

```
  1   2   3   4   5   6   7   8   9   10  11  12  13  14  15  16
H-Ser-Glu-Leu-Lys-Ser-Phe-Pro-Glu-Val-Val-Gly-Lys-Thr-Val-Asp-Gln-

 17  18  19  20  21  22  23  24  25  26  27  28  29  30  31  32  33
Ala-Arg-Glu-Tyr-Phe-Thr-Leu-His-Tyr-Pro-Gln-Tyr-Asp-Val-Tyr-Phe-Leu-

 34  35  36  37  38  39  40  41  42  43  44  45  46  47  48  49  50
Pro-Glu-Gly-Ser-Pro-Val-Thr-Leu-Asp-Leu-Arg-Tyr-Asn-Arg-Val-Arg-Val-

 51  52  53  54  55  56  57  58  59  60  61  62  63  64  65  66
Phe-Tyr-Asn-Pro-Gly-Thr-Asn-Val-Val-Asn-His-Val-Pro-His-Val-Gly-OH
```

(Siehe Schema Seite 5 ff.)

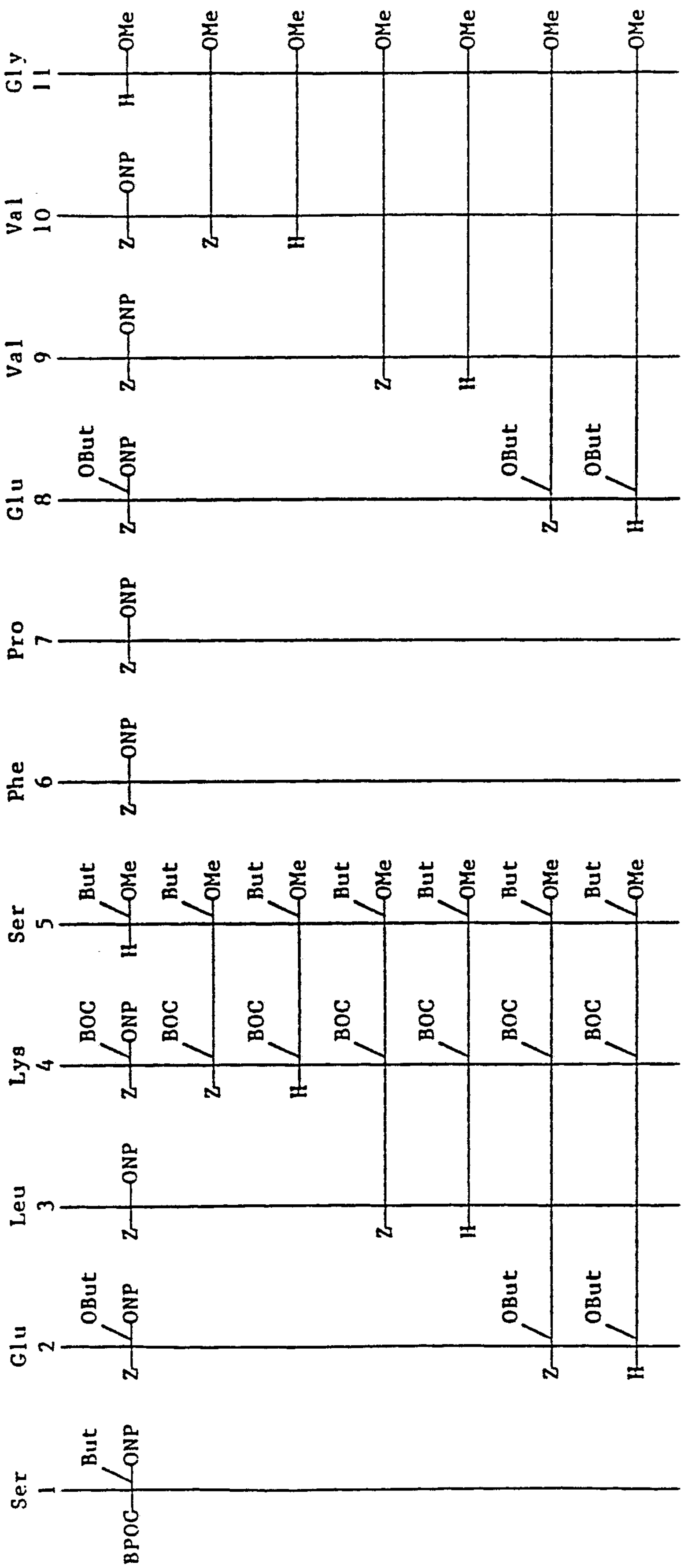


Synthese der Sequenz I, 1-11

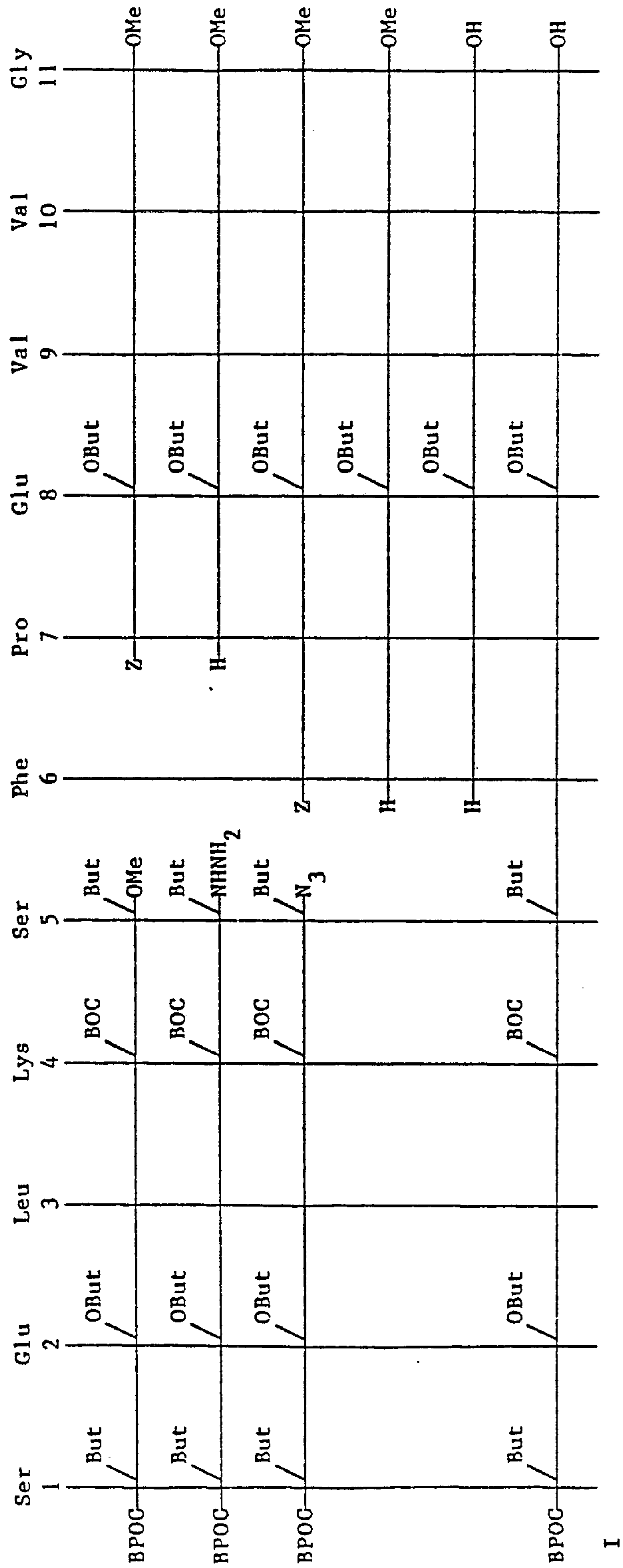

Fortsetzung der Synthese der Sequenz I, 1-11

Synthese der Sequenz II, 12-21 (Arg18 durch Protonierung geschützt)

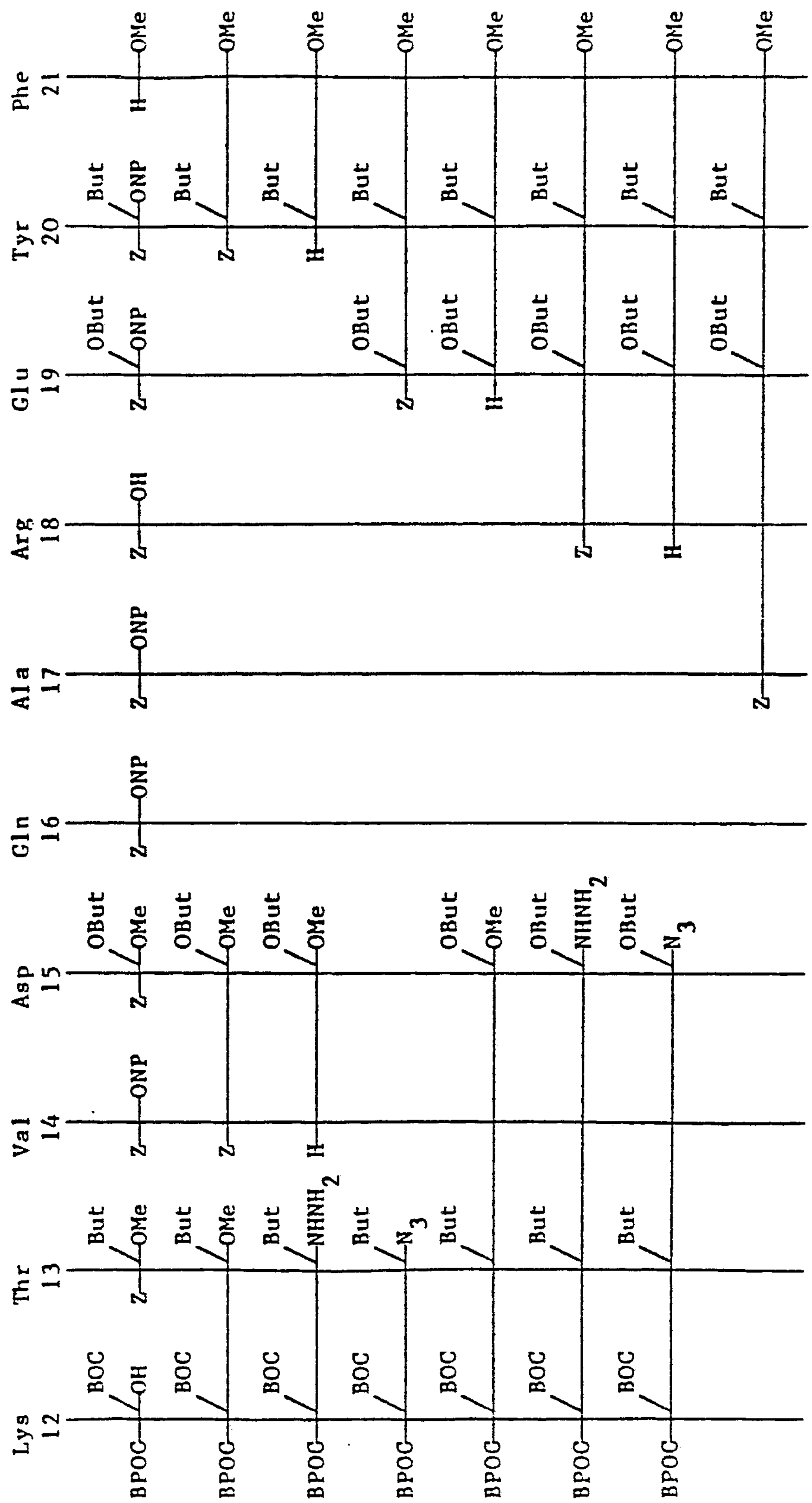

Fortsetzung der Synthese der Sequenz II, 12-21

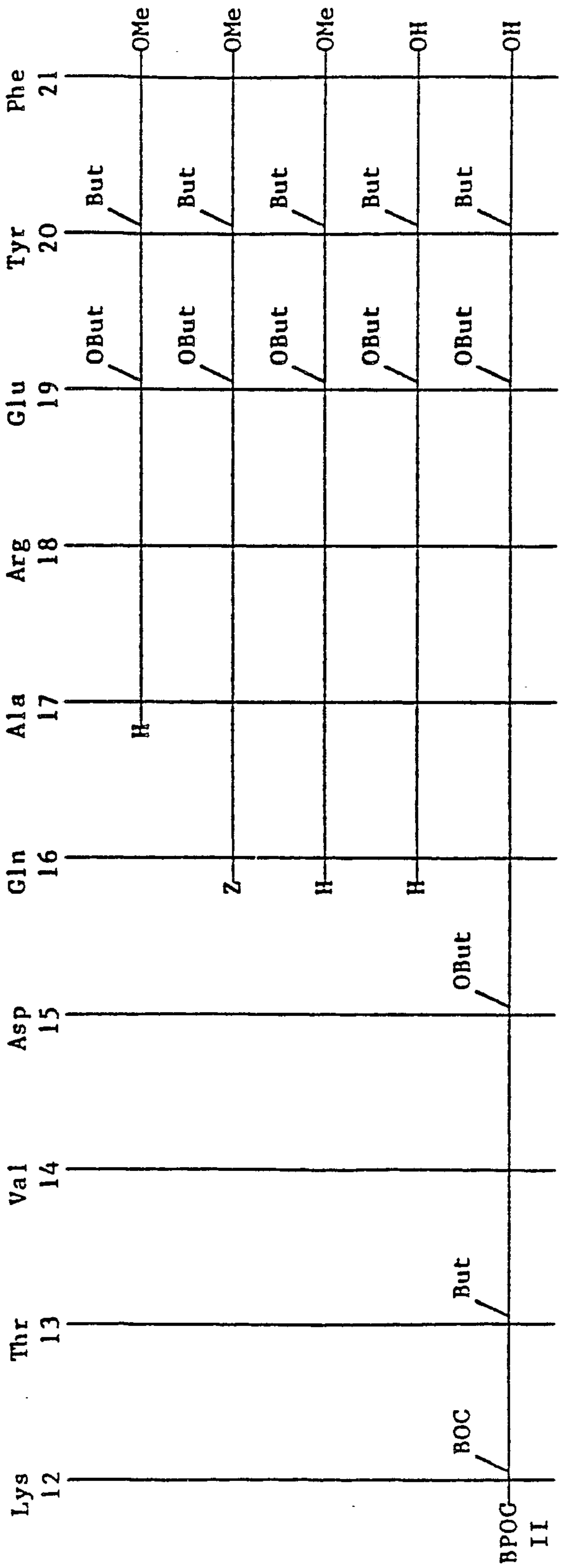

Synthese der Sequenz III, 22-29 ($His^{24}$ ungeschützt)

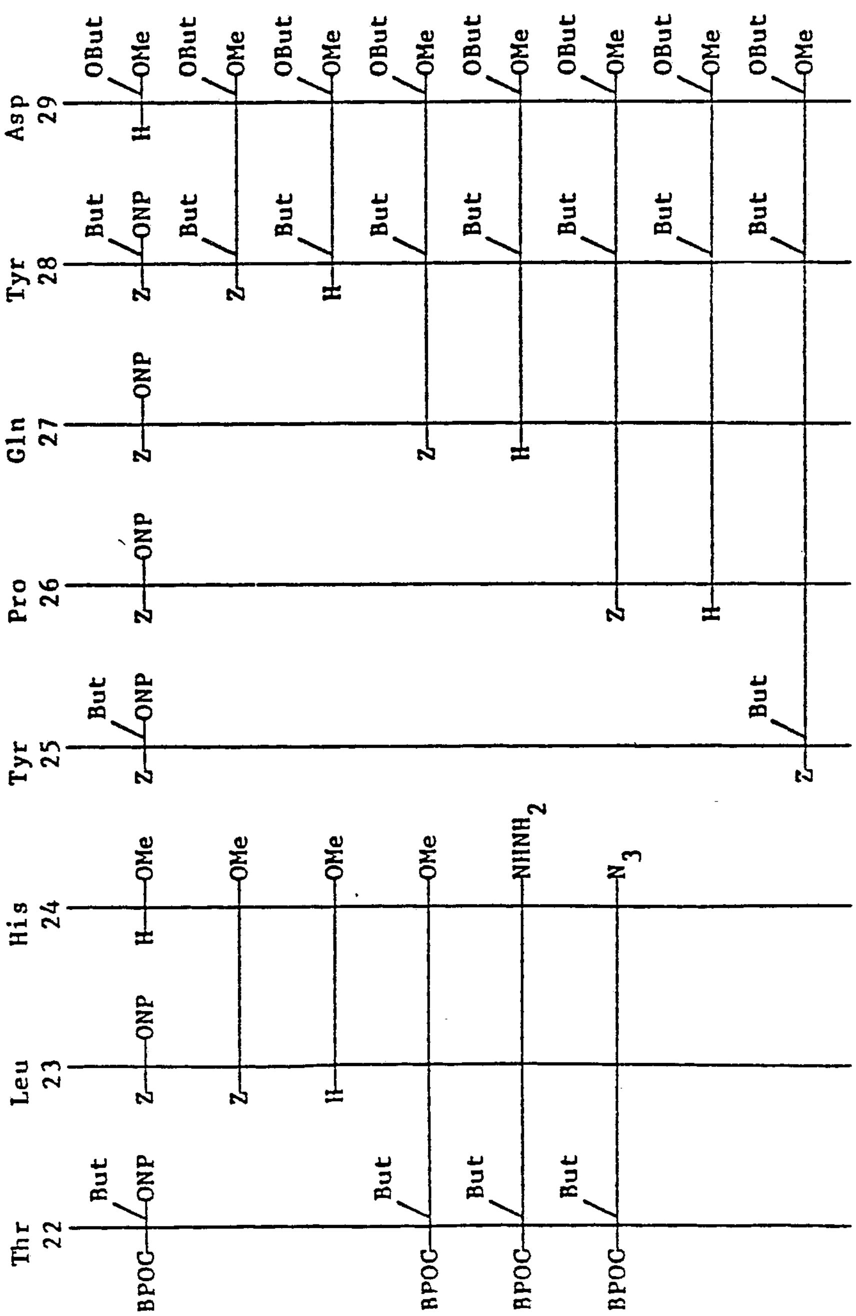

Fortsetzung der Synthese der Sequenz III, 22-29

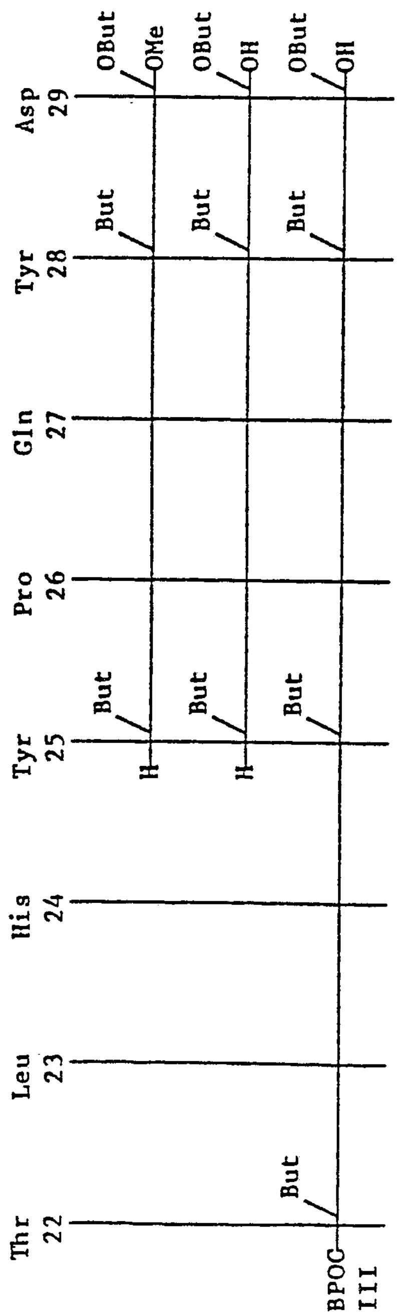

Synthese der Sequenz IV, 30-36

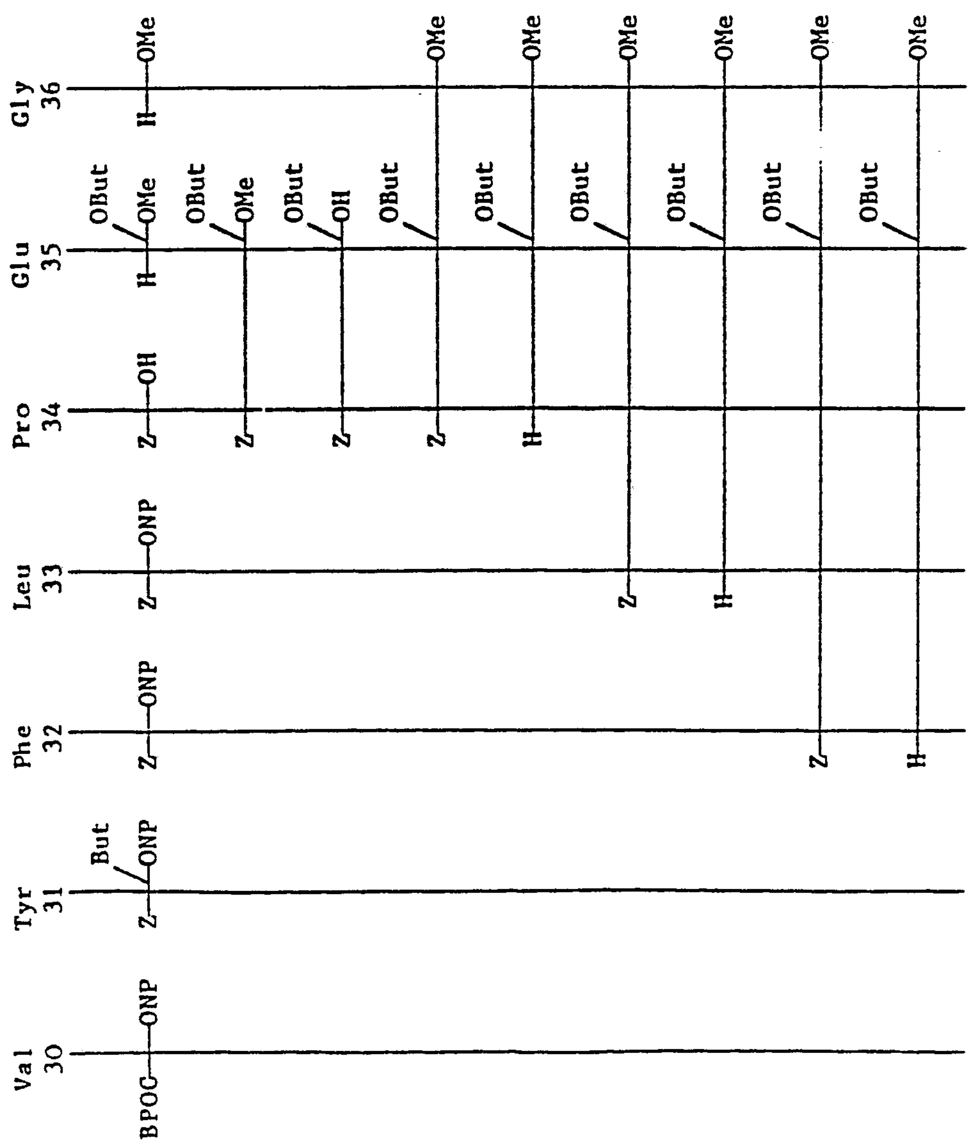

Fortsetzung der Synthese der Sequenz IV, 30-36

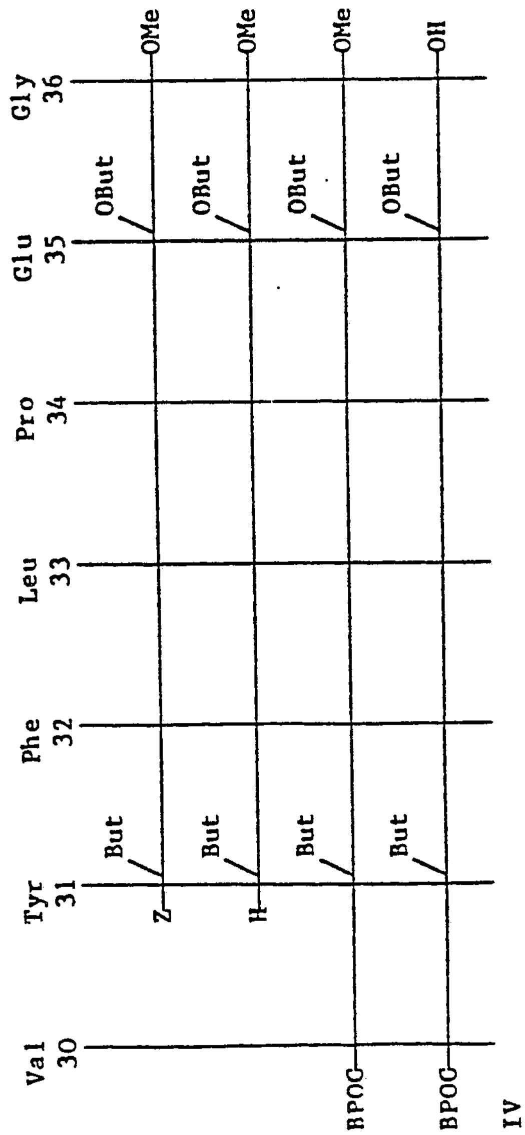

Synthese der Sequenz V, 37-45 ($Arg^{44}$ durch Protonierung geschützt)

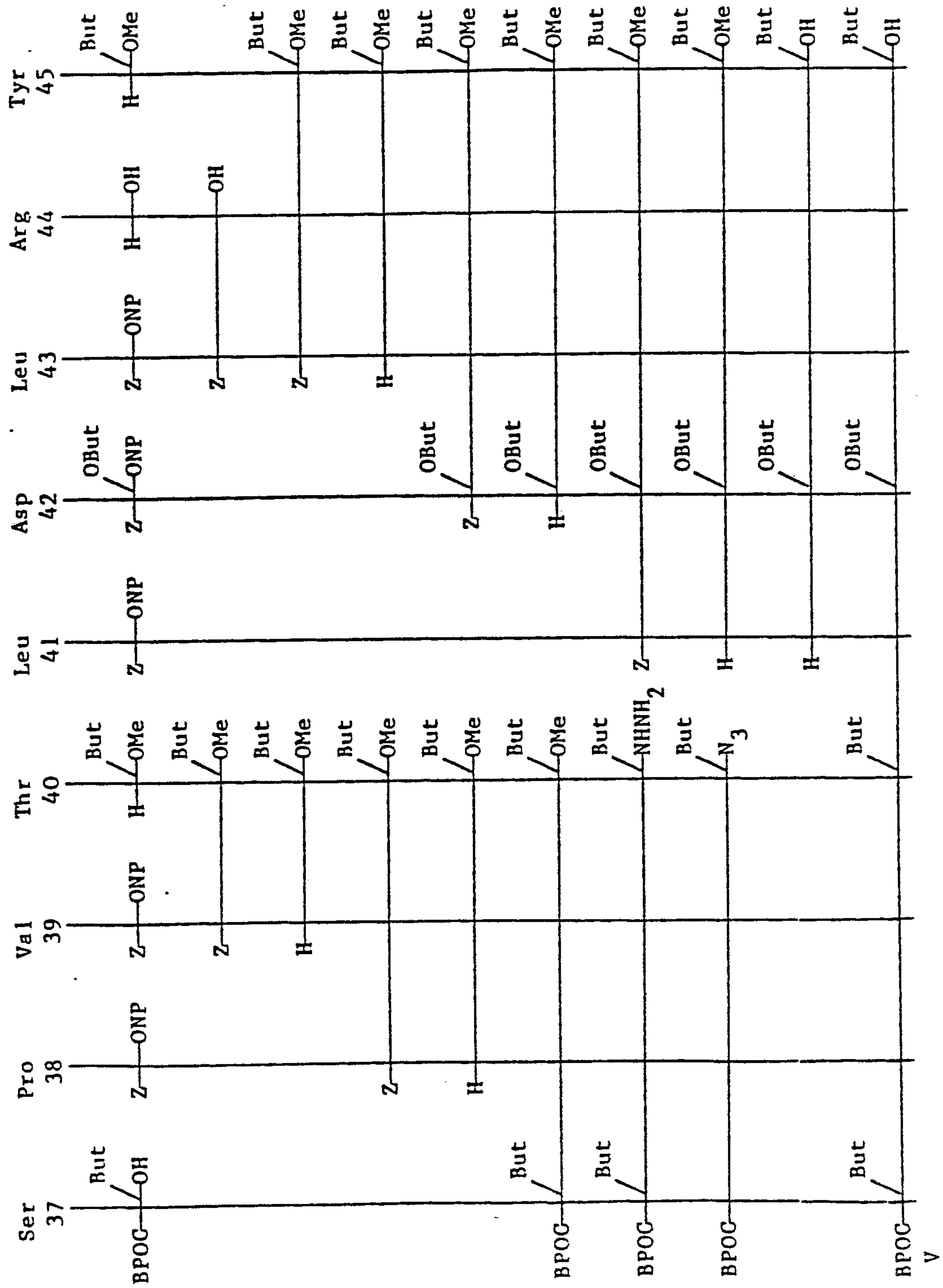

Synthese der Sequenz VI, 46-55 ($Arg^{47,49}$ durch Protonierung geschützt)

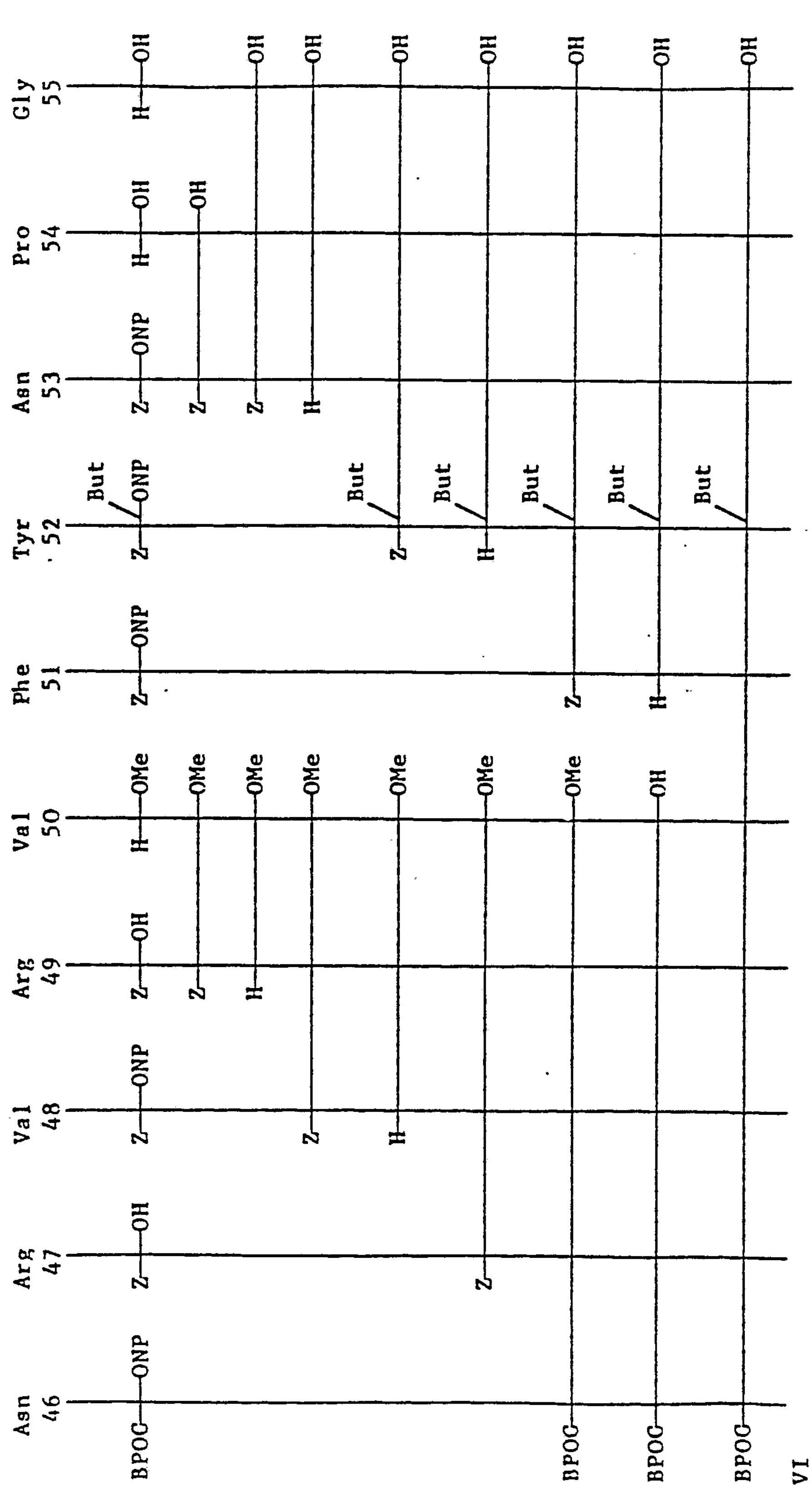

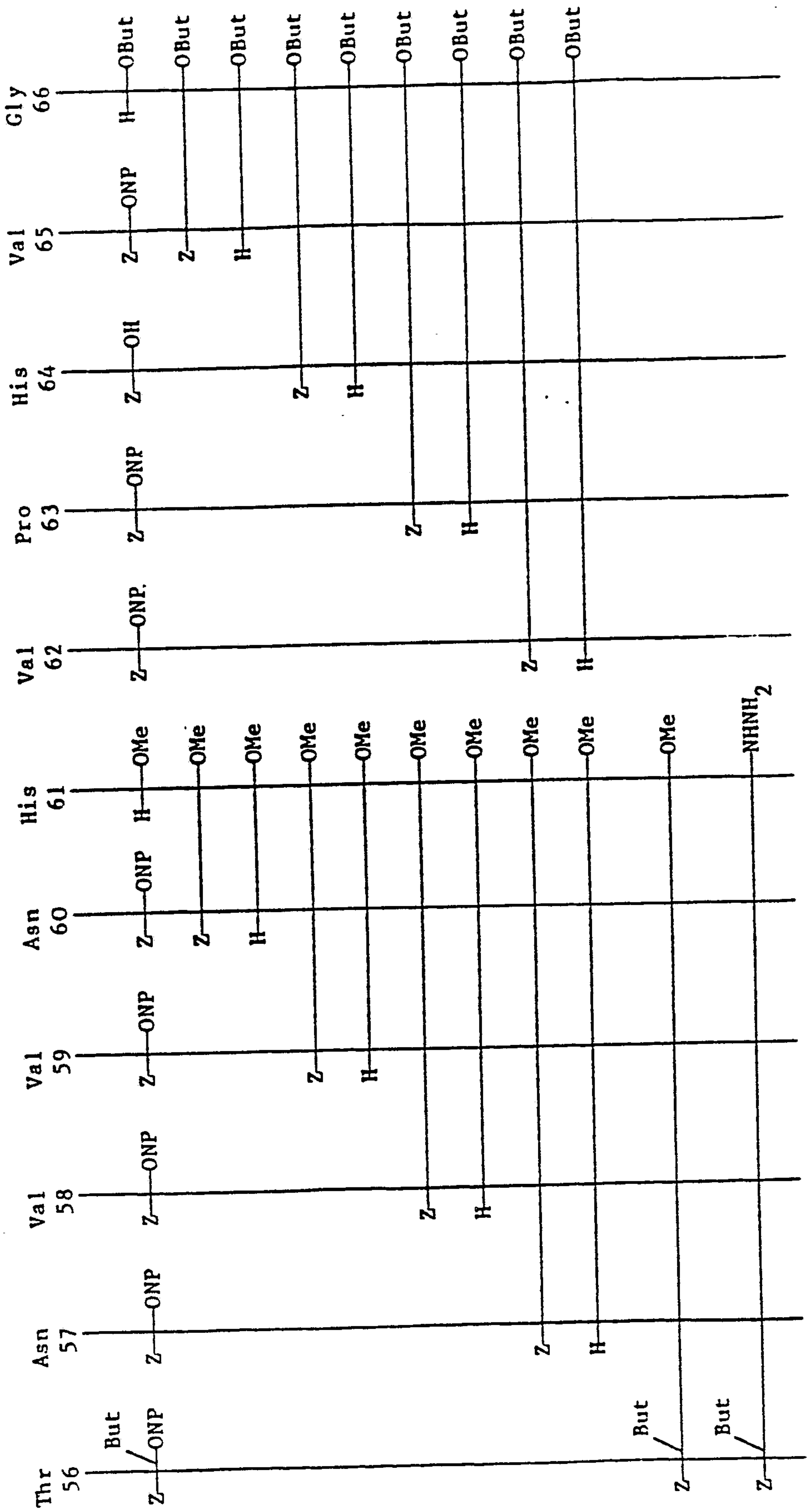

Synthese der Sequenz VII, 56-66 ($His^{61,64}$ ungeschützt)

Fortsetzung der Synthese der Sequenz VII, 56-66 ($His^{61,64}$ ungeschützt)

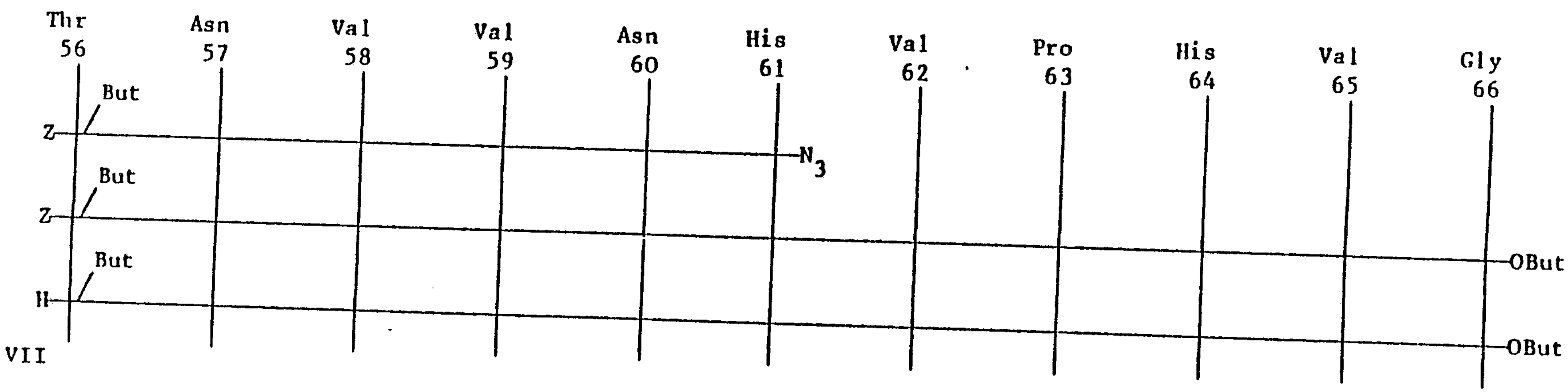

Aufbau der geschützten und freien Gesamtsequenz 1-66 aus den vorstehend beschriebenen Bruchstücken

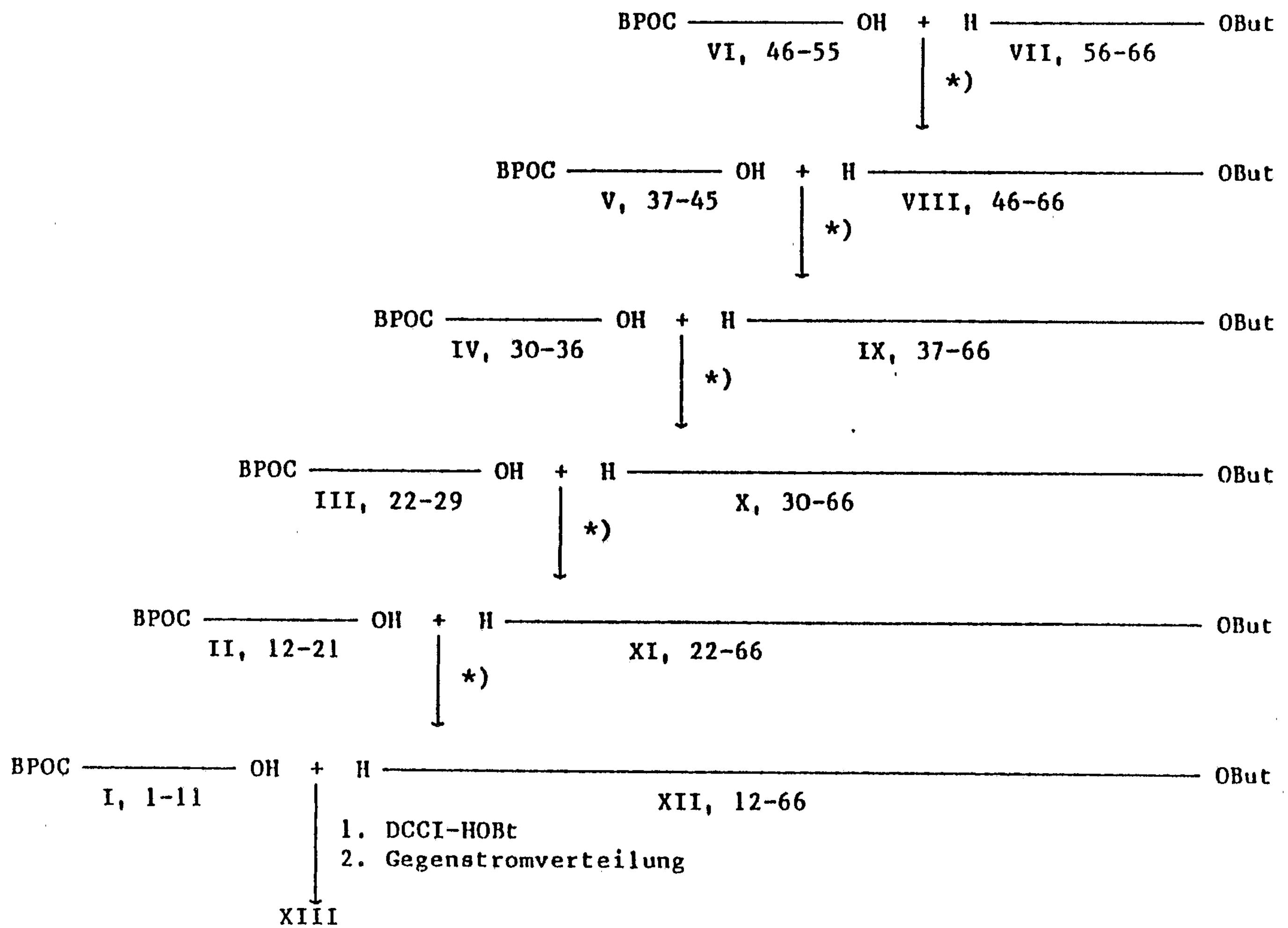


*) 1. DCCI-HOBt, 2. Gegenstromverteilung, 3. H' (pH-Stat)

1 10
XIII = BPOC-Ser(But)-Glu(OBut)-Leu-Lys(BOC)-Ser(But)-Phe-Pro-Glu(OBut)-Val-Val-Gly-Lys(BOC)-
20
Thr(But)-Val-Asp(OBut)-Gln-Ala-Arg-Glu(OBut)-Tyr(But)-Phe-Thr(But)-Leu-His-Tyr(But)-Pro-Gln-Tyr(But)-
30 40
Asp(OBut)-Val-Tyr(But)-Phe-Leu-Pro-Glu(OBut)-Gly-Ser(But)-Pro-Val-Thr(But)-Leu-Asp(OBut)-Leu-Arg-
50 60
Tyr(But)-Asn-Arg-Val-Arg-Val-Phe-Tyr(But)-Asn-Pro-Gly-Thr(But)-Asn-Val-Val-Asn-His-Val-Pro-His-Val-
Gly-OBut

↓
1. 90 % Trifluoressigsäure
2. Ionenaustauscher
Trifluoracetat gegen Acetat
3. Gegenstromverteilung

XIV = modifizierter Inhibitor F1

2. Synthese des modifizierten Protease-Inhibitors F2

1 2 3 4 5 6 7 8 9 10 11 12 13 14
H-Leu-Lys-Ser-Phe-Pro-Glu-Val-Val-Gly-Lys-Thr-Val-Asp-Gln-

15 16 17 18 19 20 21 22 23 24 25 26 27 28 29 30 31
Ala-Arg-Glu-Tyr-Phe-Thr-Leu-His-Tyr-Pro-Gln-Tyr-Asp-Val-Tyr-Phe-Leu-

32 33 34 35 36 37 38 39 40 41 42 43 44 45 46 47 48
Pro-Glu-Gly-Ser-Pro-Val-Thr-Leu-Asp-Leu-Arg-Tyr-Asn-Arg-Val-Arg-Val-

49 50 51 52 53 54 55 56 57 58 59 60 61 62 63 64
Phe-Tyr-Asn-Pro-Gly-Thr-Asn-Val-Val-Asn-His-Val-Pro-His-Val-Gly-OH

Zur Synthese dieses Wirkstoffs verwendet man die vorstehend aufgebaute Sequenz XII und kuppelt mit dem nachfolgend beschriebenen, geschützten Nonapeptid XV.

(Siehe Schema Seite 19 ff.)

Synthese des zur Herstellung des Inhibitors F2 verwendeten Nonapeptides XV, 1-9

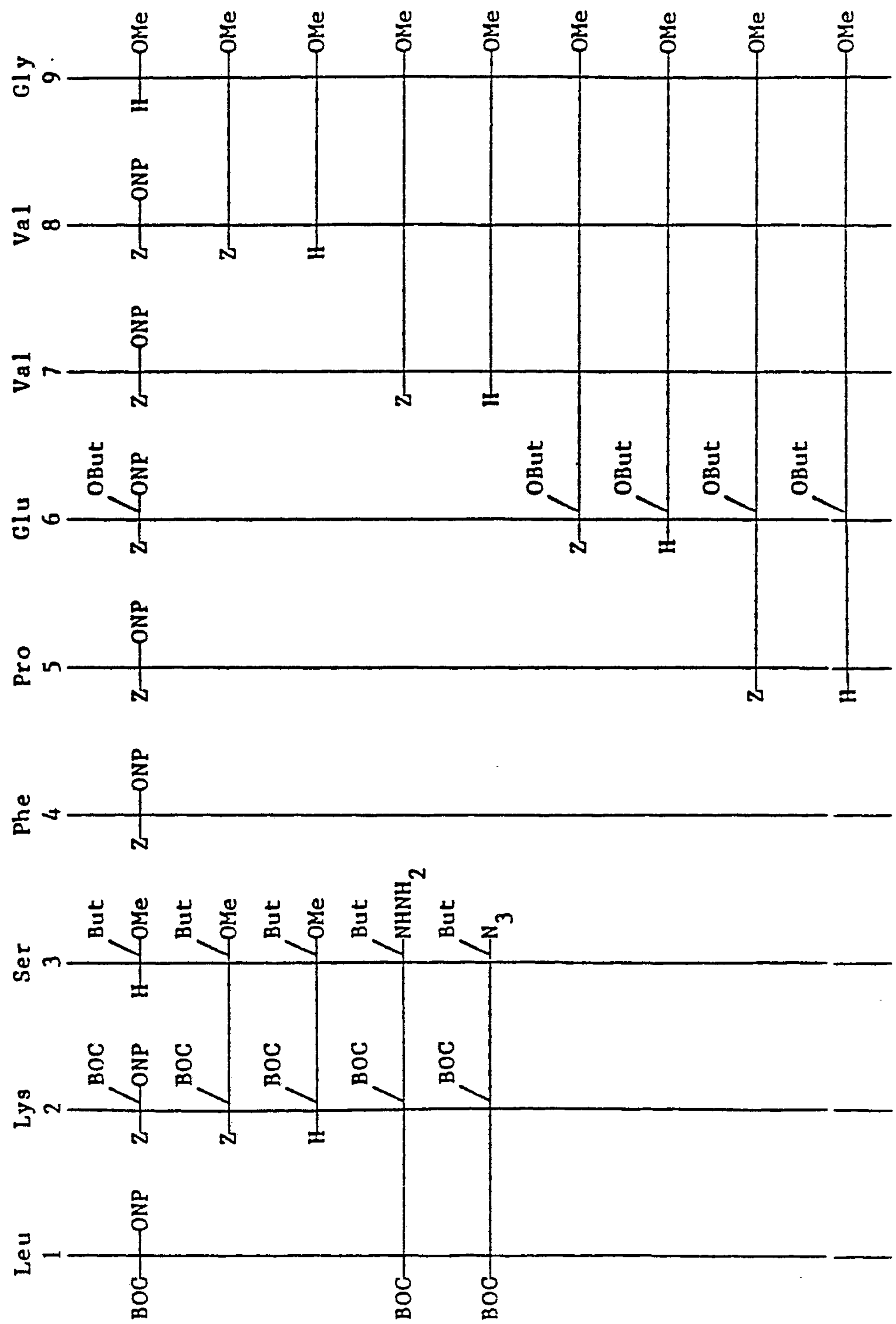

Fortsetzung der Synthese des zur Herstellung des Inhibitors F2 verwendeten Nonapeptides XV, 1-9

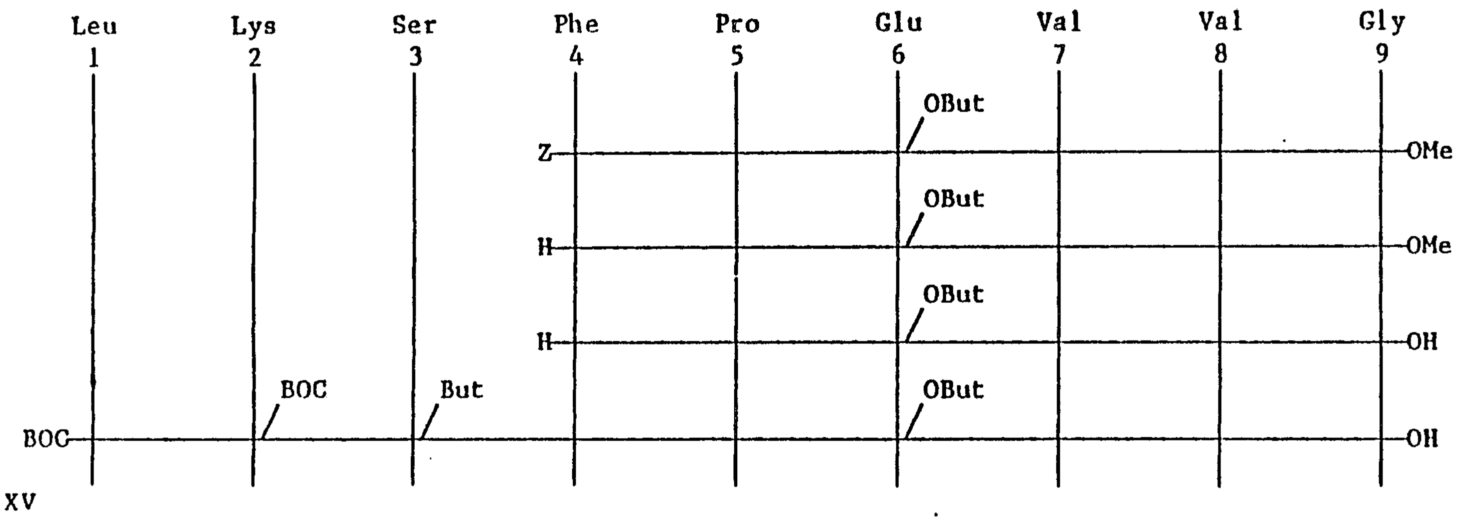

XV

Dann verfährt man wie folgt :

BOC ———— OH + H ———————— OBut
XV, 1-9 XII, 10-64

(die Zählung der 55 Aminosäurebausteine der Sequenz XII ist hier gegenüber der bei F 1 geändert, da der vollständige modifizierte Inhibitor F 2 zwei Aminosäuren weniger aufweist)

↓

XVI

1 10
XVI = BOC-Leu-Lyc(BOC)-Ser(But)-Phe-Pro-Glu(OBut)-Val-Val-Gly-Lys(BOC)-Thr(But)-Val-
20
Asp(OBut)-Gln-Ala-Arg-Glu(OBut)-Tyr(But)-Phe-Thr(But)-Leu-His-Tyr(But)-Pro-Gln-Tyr(But)-Asp(OBut)-
30 40
Val-Tyr(But)-Phe-Leu-Pro-Glu(OBut)-Gly-Ser(But)-Pro-Val-Thr(But)-Leu-Asp(OBut)-Leu-Arg-Tyr(But)-
50 60
Asn-Arg-Val-Arg-Val-Phe-Tyr(But)-Asn-Pro-Gly-Thr(But)-Asn-Val-Val-Asn-His-Val-Pro-His-Val-Gly-OBut

1. 90 % Trifluoressigsäure
2. Austausch Trifluoracetat gegen Acetat
3. Gegenstromverteilung

↓

XVII = modifizierter Inhibitor F2

Die gemäss der vorliegenden Erfindung erhältlichen modifizierten Egline weisen wertvolle pharmakologische Eigenschaften auf und können, wie die Egline (vgl. z. B. DE-OS 2 808 396), prophylaktisch oder insbesondere therapeutisch angewendet werden.

Die erfindungsgemässen neuen Verbindungen können demgemäss zur Prophylaxe und für die therapeutische Behandlung von Lungenerkrankungen, z. B. durch Leukocyten-Elastase hervorgerufene Lungenerkrankungen, wie Lungenemphysem und ARDS (« acute respiratory distress syndrome »), unter Umständen auch Mucoviscidose, ferner bei septischem Schock sowie als Antiphlogistika und Antiinflammatorika verwendet werden. Die Verwendung der erfindungsgemässen neuen Eglin-Verbindungen bei der prophylaktischen und therapeutischen Behandlung der genannten Krankheitsbilder ist ebenfalls Gegenstand der vorliegenden Erfindung.

Die Erfindung betrifft ebenfalls pharmazeutische Zusammensetzungen, welche wenigstens eine der erfindungsgemässen Verbindungen, gegebenenfalls zusammen mit einem pharmazeutisch annehmbaren Träger und/oder Hilfsstoffen, enthalten.

Diese Zusammensetzungen können insbesondere bei den oben angegebenen Indikationen Verwendung finden, wenn sie z. B. parenteral (wie intravenös oder intrapulmonal) oder topisch verabreicht werden. Die Dosierung hängt in erster Linie von der spezifischen Verarbeitungsform und vom Zweck der Therapie bzw. Prophylaxe ab.

Die Verabreichung erfolgt durch intravenöse Injektion oder intrapulmonal, durch Inhalation, z. B. mit einem Bird-Gerät. Dementsprechend enthalten pharmazeutische Präparate zur parenteralen Verabreichung in Einzeldosis-Form in Abhängigkeit von der Applikationsart pro Dosis etwa 10 bis 50 mg der erfindungsgemässen Verbindung. Neben dem Wirkstoff enthalten diese pharmazeutischen Zusammensetzungen üblicherweise noch Natriumchlorid, Mannit oder Sorbit zur Einstellung der Isotonie. Sie können in gefriergetrockneter oder gelöster Form vorliegen, wobei Lösungen mit Vorteil ein antibakteriell wirkendes Konservierungsmittel, z. B. 0,2 bis 0,3 % 4-Hydroxybenzoesäure-methylester oder -ethylester, enthalten können.

Ein Präparat für die topische Anwendung kann als wässrige Lösung, Lotion oder Gelee, ölige Lösung oder Suspension, oder fetthaltige oder insbesondere Emulsions-Salbe vorliegen. Ein Präparat in Form einer wässrigen Lösung erhält man beispielsweise dadurch, dass man die erfindungsgemässen Wirkstoffe in einer wässrigen Pufferlösung von pH 4 bis 7,5 löst und gewünschtenfalls einen weiteren Wirkstoff, z. B. ein Antiinflammatorikum, und/oder ein polymeres Haftmittel, z. B. Polyvinylpyrrolidon, und/oder ein Konservierungsmittel zufügt. Die Konzentration des Wirkstoffs beträgt etwa 0.1 bis etwa 5 mg, vorzugsweise 0,25 bis 1,0 mg, in 10 ml einer Lösung bzw. 10 g eines Gelees.

Eine ölige Applikationsform für die topische Verabreichung erhält man beispielsweise durch Suspendieren der erfindungsgemässen Wirkstoffe in einem Oel, gegebenenfalls unter Zusatz von Quellmitteln, wie Aluminiumstearat, und/oder grenzflächenaktiven Mitteln (Tensiden), deren HLB-Wert (« hydrophilic-lipophilic-balance ») unter 10 liegt, wie Fettsäuremonoester mehrwertiger Alkohole, z. B. Glycerinmonostearat, Sorbitanmonolaurat, Sorbitanmonostearat oder Sorbitanmonooleat. Eine fetthaltige Salbe erhält man z. B. durch Suspendieren der erfindungsgemässen Wirkstoffe in einer streichbaren Fettgrundlage, gegebenenfalls unter Zusatz eines Tensids vom HLB-Wert unter 10. Eine Emulsionssalbe erhält man durch Verreiben einer wässrigen Lösung der erfindungsgemässen Wirkstoffe in einer weichen, streichbaren Fettunterlage unter Zusatz eines Tensids, dessen HLB-Wert unter 10 liegt. Alle diese topischen Applikationsformen können auch Konservierungsmittel enthalten. Die Konzentration des Wirkstoffes beträgt etwa 0.1 bis etwa 5 mg, vorzugsweise 0,25 bis 1,0 mg, in etwa 10 g der Grundmasse.

Inhalationspräparate für die Behandlung der Atemwege durch intrapulmonale Verabreichung sind z. B. Aerosole oder Sprays, welche den pharmakologischen Wirkstoff in Form von Tropfen einer Lösung oder Suspension verteilen können. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, wobei diese mittels einer geeigneten Verdichtungs- und Entspannungsvorrichtung nach Bedarf erzeugt werden kann.

Besonders geeignet für die Verabreichung sind Bird-Beatmungsgeräte, welche in der Medizin eingeführt und bekannt sind ; dabei wird eine Lösung des Wirkstoffs ins Gerät gegeben und mit leichtem Ueberdruck vernebelt und in die Lunge des beatmeten Patienten gebracht.

Je nach Alter, individuellem Zustand und Art der Erkrankung beträgt bei einem Warmblüter (Menschen und Tiere) von etwa 70 kg Gewicht die Dosierung bei intrapulmonaler Verabreichung etwa 10 bis etwa 30 mg pro Inhalation (ein- bis zweimal täglich) und bei intravenöser Verabreichung, z. B. auch durch Dauerinfusion, bei etwa 10 bis etwa 150 mg pro Tag.

Therapeutisch wirksame Sputum- und Plasmakonzentrationen, welche mittels immunologischer Verfahren, wie ELISA, bestimmt werden können, liegen zwischen 10 und 200 Âg/ml.

Die folgenden Beispiele sollen die vorstehend beschriebene Erfindung illustrieren, ohne deren Umfang in irgendeiner Weise zu beschränken.

## Beispiel 1

### Herstellung des modifizierten Inhibitors F1 durch limitierte Proteolyse

Man bereitet eine Inkubationslösung, indem man eine Kathepsin C-Lösung (20 U/l · 14 ml) in 200 Âl eines Puffersystems bestehend aus 40 Âl 4 %igem Pyridin, 79 Âl Wasser, 40 Âl 0.5 %iger Essigsäure, 32 Âl 0.1 N Salzsäure, 8 Âl 0.375 M 2-Mercaptoethanol, 2 Âl 0.2 M EDTA löst. Man versetzt eine Lösung von 2.7 mg Eglin C (337 nMol) in 150 Âl 1 %iger Essigsäure mit 1 ml Kathepsin (-Inkubationslösung und stellt den Ansatz auf pH 5.0 ein. Nach 4-stündiger Inkubationszeit bei 37 °C wird der Ansatz auf einen Sephadex® SP-C25-Kationenaustauscher aufgetragen (Säule : 0.5 cm × 50 cm). Das Säulenmaterial wird mit 0.05 M Ammoniumacetat von pH 5.0 äquilibriert. 5 Stunden nach dem Auftragen wird mittels eines Gradienten von pH 5.0, hergestellt aus gleichen Volumenteilen (250 ml) 0.05 M Ammoniumacetat und 0.4 M Natriumchlorid in 0.05 M Ammoniumacetat eluiert. Der Durchfluss wird auf 1.6 ml pro Stunde eingestellt, die Fraktionsvolumina betragen 0.8 ml. Zur Entsalzung werden Fraktionen in einem Mikroultrafiltrationssystem 8MC von Amicon gegen 1 %ige Essigsäure mit einer UM2-Membran diafiltriert.

Der so erhaltene modifizierte Inhibitor F1 weist mittels der Disk-Elektrophorese (pH 8.9 ; 15 %iges Gel ; entsprechend einem Maurer-System Nr. 2) einen $R_f$-Wert von 0.42 und eine Dissoziationskonstante $K_i = 5.52 \times 10^{-11}$ mol/l auf.

Für Aminosäureanalysen werden entsalzte Aliquote 24 und 48 Stunden unter Stickstoff im Vakuum hydrolysiert und im Aminosäureanalysator (Durrum, 2 nMol Programm) analysiert. N-terminale Bestimmungen werden mit der Dansyltechnik durchgeführt. Ein 10-Schritt-Edman-Abbau wird zur eindeutigen Sequenzzuordnung durchgeführt.

## Beispiel 2

### Herstellung des modifizierten Inhibitors F2 durch limitierte Proteolyse

Die Herstellung des modifizierten Inhibitors F2 erfolgt analog dem oben beschriebenen Verfahren, jedoch wird die Reaktionslösung 48 Stunden bei 37 °C inkubiert. Bei der chromatographischen Auftrennung der Reaktionsmischung werden zuerst der modifizierte Inhibitor F1, anschliessend der modifizierte Inhibitor F2 eluiert.

Der modifizierte Inhibitor F2 weist mittels der Disk-Elektrophorese (pH 8.9 ; 15 %iges Gel ; entsprechend einem Maurer-System Nr. 2) einen $R_f$-Wert von 0.3 und eine Dissoziationskonstante $K_i = 5.37 \times 10^{-11}$ mol/l auf. Aminosäureanalysen, N-terminale Bestimmungen und Edman-Abbau zur Sequenzzuordnung wie oben bei Inhibitor F1 angegeben.

Beispiel 3

Pharmazeutisches Präparat enthaltend den modifizierten Protease-Inhibitor F1 oder F2 für parenterale Applikation

Eine gemäss Beispiel 1 oder 2 hergestellte Lösung wird gegen eine 0,9 %ige NaCl-Lösung dialysiert. Die Konzentration der Lösung wird danach durch Verdünnen mit der gleichen NaCl-Lösung auf 1 mg/ml oder 10 mg/ml eingestellt. Diese Lösungen werden durch Ultrafiltration (Membranen mit 0,22 μm Poren) sterilisiert.

Die sterilisierten Lösungen sind direkt verwendbar für die intravenöse Verabreichung, für die Dauertropfinfusion, sowie zur Vernebelung in einem Inhalationsgerät (z. B. Bird).

**Patentansprüche** (für die Vertragsstaaten : BE, CH, DE, FR, GB, IT, LI, LU, NL, SE)

1. Modifizierte Egline B oder C, dadurch gekennzeichnet, dass die Modifizierung in einer Verkürzung der Primärstruktur von Eglin B oder C um 2 bis 10 Aminosäurebausteine am Anfang des Moleküls (N-Terminus) und/oder 2 bis 6 Aminosäurebausteine am Ende des Moleküls (C-Terminus) besteht.

2. Modifizierte Egline B oder C nach Anspruch 1, dadurch gekennzeichnet, dass die Modifizierung in einer Verkürzung der Primärstruktur von Eglin B oder C um 2 bis 6 Aminosäurebausteine am Anfang des Moleküls (N-Terminus) und/oder 2 Aminosäurebausteine am Ende des Moleküls (C-Terminus) besteht.

3. Modifiziertes Eglin C nach Anspruch 1, dadurch gekennzeichnet, dass die Modifizierung in einer Verkürzung der Primärstruktur von Eglin C um 4 bis 6 Aminosäurebausteine am Anfang des Moleküls (N-Terminus) und/oder 2 Aminosäurebausteine am Ende des Moleküls (C-Terminus) besteht.

4. Ein als Verbindung F1 bezeichnetes modifiziertes Eglin C nach Anspruch 3, gekennzeichnet durch folgende Primärstruktur :

H-Ser-Glu-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

und eine Dissoziationskonstante von $K_i = 5.52 \times 10^{-11}$ mol/l für einen Chymotrypsin-Komplex (Substrat : SucAlaAlaProPhepNA).

5. Ein als Verbindung F2 bezeichnetes modifiziertes Eglin C nach Anspruch 3, gekennzeichnet durch folgende Primärstruktur :

H-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

und eine Dissoziationskonstante $K_i = 5.37 \times 10^{-11}$ mol/l (Substrat : SucAlaAlaProPhepNA) für einen Chymotrypsin-Komplex.

6. Die in den Ansprüchen 1 bis 5 genannten Verbindungen zur Verwendung als Protease-Inhibitoren.

7. Verfahren zur Herstellung von modifizierten Eglinen B oder C nach Anspruch 1, dadurch gekennzeichnet, dass man Eglin B oder C enzymatisch einer limitierten Proteolyse unterwirft oder dass man in einer den Verbindungen nach Anspruch 1 entsprechenden Verbindung, in welcher mindestens eine von vorhandenen Amino-, Hydroxy- und/oder Carboxygruppen eine Schutzgruppe trägt, vorhandene Schutzgruppen abspaltet.

8. Verfahren nach Anspruch 7, dadurch gekennzeichnet, dass man

a) Eglin B oder C in ein Puffersystem von pH 4 bis 7 einbringt,

b) mit einer, in einem Puffersystem gelösten Peptidase und gegebenenfalls mit 0.5 % Natriumlaurylsulfat versetzt,

c) 2 bis 72 Stunden bei 10 bis 40 °C inkubiert,

d) das Reaktionsgemisch chromatographisch auftrennt und

e) entsalzt.

9. Pharmazeutische Präparate enthaltend Verbindungen der Ansprüche 1 bis 5 zusammen mit einem pharmazeutisch geeigneten Trägermaterial.

10. Pharmazeutische Präparate, gekennzeichnet durch einer Gehalt an mindestens einem Protease-Inhibitor nach Anspruch 6, gegebenenfalls in Kombination mit üblichen Trägern und Zusatzstoffen.

11. Die in den Ansprüchen 1 bis 6 genannten Verbindungen zur Anwendung in einem Verfahren zur therapeutischen Behandlung des menschlichen oder tierischen Körpers.

12. Verwendung der in den Ansprüchen 1 bis 6 genannten Verbindungen zur Herstellung von pharmazeutischen Präparaten zur prophylaktischen und therapeutischen Behandlung von Lungenerkrankungen, septischem Schock und Entzündungen.

**Patentansprüche** (für den Vertragsstaat AT)

1. Verfahren zur Herstellung der modifizierten Egline B oder C, deren Modifizierung in einer

Verkürzung der Primärstruktur von Eglin B oder C um 2 bis 10 Aminosäurebausteine am Anfang des Moleküls (N-Terminus) und/oder 2 bis 6 Aminosäurebausteine am Ende des Moleküls (C-Terminus) besteht, dadurch gekennzeichnet, daß man Englin B oder C enzymatisch einer limitierten Proteolyse unterwirft oder daß man in einer den obigen Verbindungen entsprechenden Verbindung, in welcher mindestens eine der vorhandenen Amino-, Hydroxy- und/oder Carboxygruppen eine Schutzgruppe trägt, vorhandene Schutzgruppen abspaltet.

2. Verfahren nach Anspruch 1, dadurch gekennzeichnet, daß man

a) Eglin B oder C in ein Puffersystem von pH 4 bis 7 einbringt,

b) mit einer, in einem Puffersystem gelösten Peptidase und gegebenenfalls mit 0,5 % Natriumlaurylsulfat versetzt,

c) 2 bis 72 Stunden bei 10 bis 40 °C inkubiert,

d) das Reaktionsgemisch chromatographisch auftrennt und

e) entsalzt.

3. Verfahren nach Anspruch 1 oder 2, dadurch gekennzeichnet, daß man modifizierte Egline B oder C erhält, deren Modifizierung in einer Verkürzung der Primärstruktur von Eglin B oder C um 2 bis 6 Aminosäurebausteine am Anfang des Moleküls (N-Terminus) und/oder 2-Aminosäurebausteine am Ende des Moleküls (C-Terminus) besteht.

4. Verfahren nach Anspruch 1 oder 2, dadurch gekennzeichnet, daß man modifiziertes Eglin C erhält, dessen Modifizierung in einer Verkürzung der Primärstruktur von Eglin C um 4 bis 6 Aminosäurebausteine am Anfang des Moleküls (N-Terminus) und/oder 2-Aminosäurebausteine am Ende des Moleküls (C-Terminus) besteht.

5. Verfahren nach Anspruch 4, dadurch gekennzeichnet, daß man ein als Verbindung F1 bezeichnetes modifiziertes Eglin C mit folgender Primärstruktur :

H-Ser-Glu-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

und einer Dissoziationskonstante von $K_i = 5.52 \times 10^{-11}$ mol/l für einen Chymotrypsin-Komplex (Substrat : SucAlaAlaProPhepNA) erhält.

6. Verfahren nach Anspruch 4, dadurch gekennzeichnet, daß man ein als Verbindung F2 bezeichnetes modifiziertes Eglin C mit folgender Primärstruktur :

H-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH.

und einer Dissoziationskonstante $K_i = 5.37 \times 10^{-11}$ mol/l (Substrat : SucAlaAlaProPhepNA) für einen Chymotrypsin-Komplex, erhält.

7. Verfahren zur Herstellung eines pharmazeutischen Präparates, dadurch gekennzeichnet, daß man eine nach den Ansprüchen 1 bis 6 erhältliche Verbindung einem pharmazeutisch geeigneten Trägermaterial einverleibt.

**Claims** (for the Contracting States : BE, CH, DE, FR, GB, IT, LI, LU, NL, SE)

1. Modified eglins B or C, characterised in that the modification consists in a shortening of the primary structure of eglin B or C by from 2 to 10 amino acid building blocks at the beginning of the molecule (N terminus) and/or from 2 to 6 amino acid building blocks at the end of the molecule (C terminus).

2. Modified eglins B or C according to claim 1, characterised in that the modification consists in a shortening of the primary structure of eglin B or C by from 2 to 6 amino acid building blocks at the beginning of the molecule (N terminus) and/or 2 amino acid building blocks at the end of the molecule (C terminus).

3. Modified eglin C according to claim 1, characterised in that the modification consists in a shortening of the primary structure of eglin C by from 4 to 6 amino acid building blocks at the beginning of the molecule (N terminus) and/or 2 amino acid building blocks at the end of the molecule (C terminus).

4. A modified eglin C, designated compound F1, according to claim 3, characterised by the following primary structure :

H-Ser-Glu-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

and a dissociation constant of $K_i = 5.52 \times 10^{-11}$ mol/l for a chymotrypsin complex (substrate : SucAlaAlaProPhepNA).

5. A modified eglin C, designated compound F2, according to claim 3, characterised by the following primary structure :

H-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

and a dissociation constant $K_i = 5.37 \times 10^{-11}$ mol/l (substrate : SucAlaAlaProPhepNA) for a chymotrypsin complex.

6. The compounds mentioned in claims 1 to 5 for use as protease inhibitors.

7. Process for the manufacture of modified eglins B or C according to claim 1, characterised in that eglin B or C is subjected to limited proteolysis by means of enzymes, or in that in a compound, corresponding to the compounds according to claim 1, in which at least one of the amino, hydroxy and/or carboxy groups present carries a protecting group, protecting groups are removed.

8. Process according to claim 7, characterised in that

a) eglin B or C is introduced into a buffer system of pH 4 to 7,

b) a peptidase, dissolved in a buffer system, and, optionally, 0.5 % sodium lauryl sulphate are added,

c) the whole is incubated at from 10 to 40 °C for from 2 to 72 hours,

d) the reaction mixture is separated by chromatography, and

e) desalted.

9. Pharmaceutical preparations containing compounds of claims 1 to 5 together with a pharmaceutically suitable carrier material.

10. Pharmaceutical preparations, characterised by a content of at least one protease inhibitor according to claim 6, optionally in combination with customary carriers and additives.

11. The compounds mentioned in claims 1 to 6 for use in a method for the therapeutic treatment of the human or animal body.

12. Use of the compounds mentioned in claims 1 to 6 for the manufacture of pharmaceutical preparations for prophylactic and therapeutic treatment of pulmonary diseases, septic shock and inflammations.

**Claims** (for the Contracting State AT)

1. Process for the manufacture of modified eglins B or C the modification of which consists in a shortening of the primary structure of eglin B or C by from 2 to 10 amino acid building blocks at the beginning of the molecule (N terminus) and/or from 2 to 6 amino acid building blocks at the end of the molecule (C terminus), characterised in that eglin B or C is subjected to limited proteolysis by means of enzymes, or in that in a compound, corresponding to the above compounds, in which at least one of the amino, hydroxy and/or carboxy groups present carries a protecting group, protecting groups are removed.

2. Process according to claim 1, characterised in that

a) eglin B or C is introduced into a buffer system of pH 4 to 7,

b) a peptidase, dissolved in a buffer system, and, optionally, 0.5 % sodium lauryl sulphate are added,

c) the whole is incubated at from 10 to 40 °C for from 2 to 72 hours,

d) the reaction mixture is separated by chromatography, and

e) desalted.

3. Process according to claim 1 or 2, characterised in that there are obtained modified eglins B or C the modification of which consists in a shortening of the primary structure of eglin B or C by from 2 to 6 amino acid building blocks at the beginning of the molecule (N terminus) and/or 2 amino acid building blocks at the end of the molecule (C terminus).

4. Process according to claim 1 or 2, characterised in that there is obtained modified eglin C the modification of which consists in a shortening of the primary structure of eglin C by from 4 to 6 amino acid building blocks at the beginning of the molecule (N terminus) and/or 2 amino acid building blocks at the end of the molecule (C terminus).

5. Process according to claim 4, characterised in that there is obtained a modified eglin C, designated compound F1, having the following primary structure :

H-Ser-Glu-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

and a dissociation constant of $K_i = 5.52 \times 10^{-11}$ mol/l for a chymotrypsin complex (substrate : SucAlaAlaProPhepNA).

6. Process according to claim 4, characterised in that there is obtained a modified eglin C, designated compound F2, having the following primary structure :

H-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

and a dissociation constant $K_i = 5.37 \times 10^{-11}$ mol/l (substrate : SucAlaAlaProPhepNA) for a chymotrypsin complex.

7. Process for the manufacture of a pharmaceutical preparation, characterised in that a compound obtainable according to claims 1 to 6 is incorporated into a pharmaceutically suitable carrier material.

**Revendications** (pour les Etats contractants : BE, CH, DE, FR, GB, IT, LI, LU, NL, SE)

1. Egline B ou C modifiée, caractérisée en ce que la modification consiste en un raccourcissement de la structure primaire de l'égline B ou C de 2 à 10 composants acides aminés au début de la molécule (extrémité N) et/ou de 2 à 6 composants acides aminés à la fin de la molécule (extrémité C).

2. Egline B ou C modifiée selon la revendication 1, caractérisée en ce que la modification consiste en un raccourcissement de la structure primaire de l'égline B ou C de 2 à 6 composants acides aminés au début de la molécule (extrémité N) et/ou de 2 composants acides aminés à la fin de la molécule (extrémité C).

3. Egline C modifiée selon la revendication 1, caractérisée en ce que la modification consiste en un raccourcissement de la structure primaire de l'égline C de 4 à 6 acides aminés au début de la molécule (extrémité N) et/ou de 2 composants acides aminés à la fin de la molécule (extrémité C).

4. Egline C modifiée selon la revendication 3 décrite comme composé F1, caractérisée par la structure primaire suivante :

H-Ser-Glu-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

et une constante de dissociation de $K_i = 5{,}52 \times 10^{-11}$ mol/l pour un complexe de chymotrypsine (substrat : SucAlaAlaProPhepNA).

5. Egline C modifiée selon la revendication 3 décrite comme composé F2, caractérisée par la structure primaire suivante :

H-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

et une constante de dissociation $K_i = 5{,}37 \times 10^{-11}$ mol/l (substrat : SucAlaAlaProPhepNA) pour un complexe de chymotrypsine.

6. Les composés mentionnés dans les revendications 1 à 5 aux fins d'application comme inhibiteurs de protéase.

7. Procédé de préparation d'églines B ou C modifiées selon la revendication 1, caractérisé en ce qu'on soumet l'égline B ou C par un moyen enzymatique à une protéolyse limitée, ou en ce qu'on élimine les groupes protecteurs présents dans un composé correspondant aux composés selon la revendication 1, dans lequel au moins un des groupes amino, hydroxy et/ou carboxy présents porte un groupe protecteur.

8. Procédé selon la revendication 7, caractérisé en ce que

a) on introduit l'égline B ou C dans un système tampon de pH 4 à 7,

b) on mélange avec une peptidase dissoute dans un système tampon et éventuellement avec 0,5 % de laurylsulfate de sodium,

c) on fait incuber pendant 2 à 72 heures à 10 à 40 °C,

d) on sépare par chromatographie le mélange réactionnel et

e) on élimine les sels.

9. Préparations pharmaceutiques contenant des composés des revendications 1 à 5 avec un support pharmaceutique approprié.

10. Préparations pharmaceutiques caractérisées en ce qu'elles contiennent au moins un inhibiteur de protéase selon la revendication 6, éventuellement en combinaison avec des supports et additifs habituels.

11. Les composés mentionnés dans les revendications 1 à 6 aux fins d'application dans un procédé de traitement thérapeutique du corps humain ou animal.

12. Application des composés mentionnés dans les revendications 1 à 6 à la préparation de préparations pharmaceutiques pour le traitement prophylactique et thérapeutique des maladies pulmonaires, du choc septique et des inflammations.

**Revendications** (pour l'Etat contractant AT)

1. Procédé de préparation de l'égline B ou C modifiée, dont la modification consiste en un raccourcissement de la structure primaire de l'égline B ou C de 2 à 10 composants acides aminés au début de la molécule (extrémité N) et/ou de 2 à 6 composants acides aminés à la fin de la molécule (extrémité C), caractérisé en ce qu'on soumet l'égline B ou C par un moyen enzymatique à une protéolyse limitée, ou en ce que dans un composé correspondant à l'un des composés ci-dessus, où au moins l'un des groupes amino, hydroxy et/ou carboxy présents porte un groupe protecteur, on élimine les groupes protecteurs présents.

2. Procédé selon la revendication 1, caractérisé en ce que

a) on introduit l'égline B ou C dans un système tampon de pH 4 à 7,

b) on mélange avec une peptidase dissoute dans un système tampon et éventuellement avec 0,5 % de laurylsulfate de sodium,

c) on fait incuber pendant 2 à 72 heures à 10 à 40 °C,
d) on sépare par chromatographie le mélange réactionnel et
e) on élimine les sels.

3. Procédé selon la revendication 1 ou 2, caractérisé en ce qu'on obtient l'égline B ou C modifiée, dont la modification consiste en un raccourcissement de la structure primaire de l'égline B ou C de 2 à 6 composants acides aminés au début de la molécule (extrémité N) et/ou de 2 composants acides aminés à la fin de la molécule (extrémité C).

4. Procédé selon la revendication 1 ou 2, caractérisé en ce qu'on obtient l'égline C modifiée, dont la modification consiste en un raccourcissement de la structure primaire de l'égline C de 4 à 6 composants acides aminés au début de la molécule (extrémité N) et/ou de 2 composants acides aminés à la fin de la molécule (extrémité C).

5. Procédé selon la revendication 4, caractérisé en ce qu'on obtient l'égline C décrite comme composé F1 ayant la structure primaire suivante :

H-Ser-Glu-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

et une constante de dissociation de $K_i = 5,52 \times 10^{-11}$ mol/l pour un complexe de chymotrypsine (substrat : SucAlaAlaProPhepNA).

6. Procédé selon la revendication 4, caractérisé en ce qu'on obtient une égline C modifiée décrite comme composé F2 ayant la structure primaire suivante :

H-Leu-Lys-Ser-Phe-Pro-Glu-Val-Val-Gly-Lys-Thr-Val-Asp-Gln-Ala-Arg-Glu-Tyr-Phe-Thr-Leu-His-Tyr-Pro-Gln-Tyr-Asp-Val-Tyr-Phe-Leu-Pro-Glu-Gly-Ser-Pro-Val-Thr-Leu-Asp-Leu-Arg-Tyr-Asn-Arg-Val-Arg-Val-Phe-Tyr-Asn-Pro-Gly-Thr-Asn-Val-Val-Asn-His-Val-Pro-His-Val-Gly-OH

et une constante de dissociation $K_i = 5,37 \times 10^{-11}$ mol/l (substrat : SucAlaAlaProPhepNA) pour un complexe de chymotrypsine.

7. Procédé de préparation d'une préparation pharmaceutique, caractérisé en ce qu'on incorpore un composé que l'on peut obtenir selon les revendications 1 à 6 à un support pharmaceutiquement approprié.